# EUROPEAN PATENT SPECIFICATION

(11) **EP 3 131 919 B1**
(45) Date of publication and mention of the grant of the patent: **10.10.2018**
(21) Application number: 15717868.2
(22) Date of filing: 17.04.2015
(51) Int. Cl.: C07K 14/245, C12N 15/62, C12N 15/70

(54) **VECTOR AND METHOD FOR EXPRESSING MOLECULES OF INTEREST IN A BACTERIAL CELL**
VEKTOR UND VERFAHREN ZUR EXPRESSION VON MOLEKÜLEN VON INTERESSE IN EINER BAKTERIELLEN ZELLE
VECTEUR ET PROCÉDÉ POUR L'EXPRESSION DE MOLÉCULES D'INTÉRÊT DANS UNE CELLULE BACTÉRIENNE

(30) Priority: 17.04.2014 EP 14165102
(43) Date of publication of application: 22.02.2017
(73) Proprietor: Universität zu Köln, 50923 Köln (DE)
(72) Inventor: CHMIELEWSKI, Markus, 50739 Köln (DE); ABKEN, Hinrich, 56414 Meudt (DE)
(74) Representative: Gramm, Lins & Partner Patent- und Rechtsanwälte PartGmbB
(86) International application number: PCT/EP2015/058400
(87) International publication number: WO 2015/158899

(56) References cited:
- EP-A1- 1 873 251
- WO-A1-01/05978
- WO-A1-99/57276
- WO-A2-02/04496
- MOHAMMAD REZA ASADI KARAM ET AL: "Assessment of immune responses of the flagellin (FliC) fused to FimH adhesin of Uropathogenic Escherichia coli", MOLECULAR IMMUNOLOGY, vol. 54, no. 1, 1 May 2013 (2013-05-01), pages 32-39, XP055140881, ISSN: 0161-5890, DOI: 10.1016/j.molimm.2012.11.002 cited in the application
- KARAM ASADI ET AL: "Cloning of fimH and fliC and expression of the fusion protein FimH/FliC from Uropathogenic Escherichia coli (UPEC) isolated in Iran.", IRANIAN JOURNAL OF MICROBIOLOGY, vol. 4, no. 2, 1 June 2012 (2012-06-01), pages 55-62, XP055139984, ISSN: 2008-3289
- SAVAR NASTARAN SADAT ET AL: "In silicoandin vivostudies of truncated forms of flagellin (FliC) of enteroaggregativeEscherichia colifused to FimH from uropathogenicEscherichia colias a vaccine candidate against urinary tract infections", JOURNAL OF BIOTECHNOLOGY, vol. 175, 12 February 2014 (2014-02-12), pages 31-37, XP028631076, ISSN: 0168-1656, DOI: 10.1016/J.JBIOTEC.2014.01.037

## Description

The present invention relates in a first aspect to a vector nucleic acid suitable for expressing at least one molecule of interest in a prokaryotic cell, in particular bacteria, and on the surface thereof, eventually released by said cell into the supernatant. In particular, the present invention relates to a vector nucleic acid comprising a nucleic acid sequence encoding a Fim polypeptide fused with a molecule of interest wherein the Fim polypeptide is Fim H, A, F, or G, in particular Fim H. In addition, the present invention relates to a cell containing said vector as well as isolated recombinant protein containing at least one module being a Fim polypeptide wherein the Fim polypeptide is Fim H, A, F, or G, in particular Fim H and a further module being a molecule of interest. In addition, the present invention relates to methods for the production of the molecule of interest using the vector nucleic acid according to the present invention. Finally, the present invention provides a kit of system containing said vector nucleic acid in particular for the production of recombinant molecule or interest.

### Prior art

The expression and production of recombinant proteins in bacterial cells is a long standing and well known procedure for obtaining recombinant proteins. A preferred host cell is the *Escherichia coli* (E. coli) bacterium. An expression system is a combination of a vector, namely an expression vector, and a host whereby said host allows the expression of the gene present in the expression vector in the host. The expression can be permanent or may be regulated by induction allowing transient or switching on and off of the expression accordingly. Expression systems are used for the expression of recombinant proteins starting from research to the industrial scale to produce reagents suitable for diagnostics and therapy.

Various expression systems are known in the art depending on the desired purpose and the host cell. Most often procaryotic cells, mostly bacteria, are used as host cells since they can be easily genetically manipulated, however, also eukaryotic expression systems for use in plants, insects and mammalian cells are described and established. Typically, the expression vector comprises a multiple cloning site allowing the incorporation of the desired molecule to being expressed, also identified as the molecule of interest (MOI). In addition, the expression vector contains suitable promoter sequences allowing transcription of the nucleic acid sequence present in the vector system. The expression vector may be an expression vector allowing for extrachromosomal expression or may be an expression vector allowing integration of the nucleic acid sequence to be expressed into the genome of the host.

The expression vector is obtained by genetic engineering and transformation of the bacteria is effected by various methods.

Although the production of recombinant proteins in expression systems using bacterial cells as host cells is well known in the art, the process is limited for various reasons. For example, expression in bacterial systems is limited due to cytoplasmic or periplasmic accumulation of the protein of interest, e.g. in inclusion bodies. Actually, one of the main disadvantages of the bacterial expression system is the accumulation of the expression product in the periplasmatic space or in inclusion bodies, thus, the dissolution of the desired expression product is not given. Various attempts have been made in the art to overcome the problem of accumulation of the expression product in the periplasmatic space and the inclusion bodies and, in addition, allowing secretion of the expression product in the culture medium. For review: Pines O, Inouye M. Expression and secretion of proteins in E. coli., Mol Biotechnol. 1999 Aug; 12(1):25-34.

To increase solubility of the recombinant expression products, it is tried to overcome the formation of inclusion bodies, e.g. using expression systems containing GST (Glutathione-S-Transferase) or N utilizations substance A (NusA). Alternatively, it is tried to direct the expression proteins into the periplasma. However, expressions systems allowing direct expression and equitation of the expression product into the extracellular environment are seldom.

A commercial product of Wacker Chemie AG, ESETEC is an expression system allowing for secretion of native recombinant protein products into the supernatant simplifying further purification steps. However, the system is based on leakage of the expression system, thus, the MOI is secreted only due to leakage of the system but the system does not allow to express a fusion protein on the surface and, eventually, releasing the MOI from the expression cells.

Expression systems for proteins of interest in gram-negative bacteria are so far based on C-terminal fusion or integration within the bacterial carrier protein. In E. coli, the outer membrane OmpA, LamB, PhoE have been used to display peptides or proteins on the cell surface (Etz, et al., J Bacteriol. 183:6924-35, 2001). In Salmonella, the Omp bacterial transporter was used to express a protein of interest (Massa, et al., Blood, 2013, 122:705-714). However, insertion of peptides longer than 60 amino acids was shown to perturb the conformation of LamB and PhoE (Agterberg, et al, Gene 88:37-45, 1990; Charbit et al., Gene 70:181-9, 1988), resulting in interference with proper cell surface localization. The OmpA expression system is additionally limited in the use of hydrophobic segments or inserts with distinct secondary structures (Ho-bom et al., Dev Biol Stand. 84:255-62, 1995).

So far, the fimbrial protein FimH has been used to display peptides incorporated within the bacterial carrier protein and thereby limited the potential size of the integrated DNA. FimH belongs to the group of adhesin proteins.

Adhesins are cell surface components or appendages of bacteria that facilitate bacterial adhesion or adherence to other cells or to inanimate surfaces. Adhesion represents an essential step in bacterial pathogenesis or infection required for colonising a new host. In gram-negative bacteria fimbria function as adhesion structures. That is, to effectively achieve adherence to host surfaces, many bacteria produce multiple adherence factors called adhesins. A typical structure of the bacteria adhesion is that of fimbria. In contrast to pili, fimbria are not involved in the exchange of genetic material, however, fimbria are often called type-1-pili. Gram-negative pathogens commonly interact with their environment using long, linear, surface-exposed protein appendages. In uropathogenic Escherichia coli, type-1 pili carry at their distal end a dedicated mannose-specific adhesin, Fim H, that is responsible for the attachment of bacteria to the bladder epithelium and their subsequent internalization and biofilm-like organization inside the urothelial cells. Type-1 pili are representative of a large class of non-covalently linked fibres on the surface of gram-negative bacteria, synthesized via the conserved chaperone/usher pathway. Type-1 pili are composed of four different subunit types (FimH, FimG, FimF and FimA).

In Gram-negative bacteria many adhesins are displayed on the bacterial surface by chaperone-usher-assisted transport, an illustrative example being type- 1 pili. A typical type-1 fimbriated bacterium has 200-500 peritrichously arranged pili on the surface. A single pilus is composed of four building elements that are added to the base of a growing organelle. Approximately 1.000 copies of the major structural component, FimA, are polymerized into a righthanded helical structure, which additionally contains small quantities of the minor components, FimF, FimG, and FimH. Hence, type-1 pili produced by gram-negative bacteria like E.coli are multi-subunit fibres crucial in the recognition of and the adhesion to host cells. Type 1 fimbria are composed of four different subunit types (Fim H, Fim G, Fim F and Fim A). The ad-hesin Fim H and the two linker subunits Fim G and Fim F form a short flexible fibrillar tip that is attached to an extended rigid and helically wound rod of Fim A subunits.

Translocation of the structural components across the inner membrane is mediated by the Sec-dependent pathway. The minor components, notably Fim H are of paramount importance for the initiation of organelle formation and determines the organelle length and number in a dose-dependent manner.

Fim H is one of the best characterized bacterial adhesin. This adhesin is responsible for D-mannose sensitive adhesion. Mature Fim H is displayed on the bacterial surface as a component of the type-1 pili.

Fim H is involved in modulating the immune response against pathogens. For example Fim H overcomes the antibody based immune response by natural conversion from the high to the low affinity state. Through this conversion, Fim H adhesion may shed the antibodies bound to it. Vaccines were based on adhesins because they are often essential to infection and are surface located, making them readily accessable to antibodies.

Taken together, Fim H is of interest for biotechnological production of proteins. Fim H adhesion proteins and methods of use are described, e.g. Fim H adhesion proteins and methods of use are described e.g. in WO 02/04496. Therein the bacterial adhesin proteins and active fragments thereof, in particular, for use in vaccine composition are disclosed.

Pallesen et. al., Microbiology, 1995, 141, 2839-2848, describe a chimeric FimH adhesion type-1 pili useful as a bacterial surface display system for heterologous sequences. Therein heterologous sequences are incorporated into the FimH protein positioned in the C-terminal domain of FimH. It is identified therein, that the C-terminal domain of FimH is amenable for integration of heterologous inserts of substantial size. Further, it is speculated therein that the chimeric FimH proteins can be used for presentation of immunologic relevant epitopes. Moreover other possibilities include protein-protein interaction and receptor recognition in general. Of note, this document identifies integrated expression of the heterologous sequence, however, expression of a protein of interest on the bacteria surface or purification of the heterologous products is not envisaged. In Asadi Karam MR et.al., Iranian J Microbiology, 2012, 4(2), 55-62, cloning of FimH and fliC and expression thereof as fusion protein is described. However, the fusion protein in the described fashion is not suitable for expression on the surface of the bacteria or for secretion into the culture medium. Similar approaches are described in Karam et. Al., Mol. Immunol, 2013, 54, 32-39 and Savar et al., J. of Biotechnology, 2014, 175, 31-37. However, the systems described therein are based on fusion proteins wherein FimH is located at the N-terminus. Further, the systems do not describe any expression of the fusion proteins on the surface of the cells but enrichment occurs in the periplasmatic space only. For purification purposes the cells are isolated from the cells by known means. Further, none of these documents identify that a leader sequence is required at the N-terminus of the MOI.

### Brief summary of the invention

In a first aspect, the present invention provides a vector nucleic acid suitable for expressing at least one molecule of interest (MOI) in a prokaryotic cell and on the surface of a prokaryotic cell, in particular bacteria, or released by the said cell, comprising in frame form 5' to 3' a nucleic acid encoding a leader sequence, a multiple cloning site for introduction of the nucleic acid sequence encoding said MOI and a nucleic acid sequence encoding a mature Fim polypeptide wherein the Fim polypeptide is Fim H, A, F, or G, in particular Fim H.

In a further aspect, the present invention provides a vector nucleic acid suitable for expressing at least one molecule of interest in a procaryotic cell and the surface of the said cell, or released by the said cell, comprising a nucleic acid sequence encoding in frame from 5' to 3' a nucleic acid encoding a leader sequence, a nucleic acid sequence encoding a molecule of interest and a nucleic acid sequence encoding a Fim polypeptide wherein the Fim polypeptide is Fim H, A, F, or G, in particular Fim H, optionally having a linker nucleic acid sequence located between the nucleic acid sequence encoding a molecule of interest and the nucleic acid sequence encoding a mature Fim polypeptide, wherein the Fim polypeptide is Fim H, A, F, or G, in particular Fim H. Said optionally present linker is a linker which does not change the reading frame of the nucleic acid sequence.

Further, the present invention provides a cell containing the vector nucleic acid according to the present invention. The cell contains said vector nucleic acid according to the present invention either stably integrated in the host or present as a minicircle expression vector for a stable or transient expression.

Moreover, an isolated recombinant protein containing at least a first module being a Fim polypeptide wherein the Fim polypeptide is Fim H, A, F, or G, in particular Fim H, and a second module being a molecule of interest, like a protein of interest, is provided.

In addition, the present invention provides a method for production of a molecule of interest using the vector nucleic acid according to the present invention for transforming a prokaryotic cell.

In addition, the present invention provides a method for screening for a molecule of interest using the vector nucleic acid according to the present invention for displaying the molecule of interest on the surface of a prokaryotic cell and using that transformed cell for screening.

Finally, the present invention relates to a kit or system containing the vector nucleic acid according to the present invention, in particular, for use in the production of recombinant molecules.

### Brief description of the drawings

Fig. 1: Figure 1 is a schematic model of the synthesis and transport of the fusion protein GlucFimH in bacterial cells. Shown is the pilus where the fusion protein is present on the outer surface of the bacteria. The fusion protein is eventually released into the extracellular space.
Fig. 2: Figure 2 shows the structure of a vector nucleic acid according to the present invention.
Fig.3: Figure 3 is an analysis of transformed bacteria cells. Shown are the flow cytometry data of wild type bacteria or transformed bacteria which express the fusion protein on the surface.
Fig. 4: Figure 4 shows the luciferase light signals of modified bacteria *in vitro* and in a pancreatic cancer mouse model.
Fig. 5: Figure 5 demonstrates that the expression of a MOI moiety depends on the integration site of the FimH molecule. The upper part shows the expression cassette while the lower part demonstrates that after induction of expression, no expression can be observed at the surface of the transformed bacterial cell.
Fig. 6: Figure 6 shows the POI expression cassette with the integrated HRV 3C protease cleavage site.
Fig. 7: Figure 7 shows the release of the recombinant MOI (POI) into the supernatant after cleavage.

### Detailed description of the present invention

In a first aspect, the present invention relates to a vector nucleic acid suitable for expressing at least one molecule of interest in a prokaryotic cell and on the surface of that cell, or for release of the product by said cell in frame from 5' to 3' a nucleic acid encoding a leader sequence, a multiple cloning site for introduction of the nucleic acid sequence encoding said MOI and a nucleic acid sequence encoding a mature Fim polypeptide wherein the Fim polypeptide is Fim H, A, F, or G, in particular Fim H.

In this connection, the term "comprise" and "comprising" as well as "contain" and "containing" includes the embodiments of "consist" and "consisting of". That is, the terms "comprising" and "comprises" and "comprised of" are synonymous with "including", "includes" or "containing", "contains", and are inclusive or open ended and do not exclude additional, non recited members, elements or method steps.

As used herein, the singular forms "a", "an" and "the" include both singular and plural reference unless context clear dictates otherwise.

Unless otherwise defined, all terms used in disclosing the invention, including technical and scientific terms, have the meaning as commonly understood by one of the ordinary skilled in the art to which this invention belongs. By means of further guidance, term definitions are included to better appreciate the teaching of the present invention.

All references cited in the present application are hereby incorporated by reference in their entirety. In particular, the teachings of all references herein specially referred to are incorporated by reference.

The term "vector nucleic acid" refers to a nucleic acid sequence present in form of a vector, typically, an expression vector which is usually a plasmid designed for expression in host cells, in particular, bacterial cells. The vector is used to introduce a specific gene into a host cell and can allow protein synthesis based on the specific gene introduced to produce a recombinant protein encoded by said gene. The vector nucleic acid is an engineered vector containing regulatory sequences. Said regulatory sequences can include enhancer and promoter regions allowing a transient or induced transcription of the gene. Said vector nucleic acid is designed to be transformed or transfected into the host cell for protein synthesis. Said vector nucleic acid may be designed to have elements allowing insertion of the nucleic acid sequence into the host chromosome. Alternatively, said vector nucleic acid may contain elements allowing episomal replication. The skilled person is well aware of vector nucleic acid sequences suitable according to present invention. The vector nucleic acid may be obtained by genetic engineering or by chemical synthesis.

The vector nucleic acid sequence according to the present invention contains a nucleic acid sequence encoding a Fim polypeptide wherein the Fim polypeptide is Fim H, A, F, or G, in particular Fim H. Said nucleic acid sequence encoding the Fim polypeptide contains a multiple cloning site for introduction of the nucleic acid sequence encoding the molecule of interest (MOI) 5' to the reading frame of the mature Fim polypeptide wherein the Fim polypeptide is Fim H, A, F, or G, in particular Fim H. That is, the multiple cloning site (MCS) is located in frame between the nucleic acid encoding a leader sequence and the nucleic acid sequence encoding the mature polypeptide.

As used herein, the term "Fim polypeptide" refers to a Fim protein described in the art derived from bacterial cells, in particular, stemming from E.coli. The Fim polypeptide is a Fim H, Fim G, Fim F, or Fim A protein. The term "Fim polypeptide" includes embodiments wherein the Fim polypeptides are equivalents of the naturally occurring Fim proteins having the same functionality, eg. fragments of said polypeptides.

As used herein, the term "leader sequence" refers to a sequence directing the molecule composed of the MOI and the mature Fim polypeptide to a predetermined compartment of the host cell, e.g. in type-1 pili on the bacterial surface. In an embodiment of the present invention, the leader sequence is the Fim leader sequence, e.g. the Fim H leader sequence. For example, the FimH leader sequence encodes a 21 amino acid peptide (e.g Seq. ID. No. 1) that is responsible for leading the matured protein of interest to the place of destination. Other Fim leader sequences may alternatively be used.

In contrast the Fim promoter is responsible for the RNA expression of the entire Fim protein including the leader.

In an embodiment, the present invention provides an expression cassette for expressing a molecule of interest in a "procaryotic cell" under the control of a promoter which may be different to the FimH promoter, in particular of the T7 bactoriaphage promoter.

Further, as used herein, the term "mature" refers to a polypeptide not containing any leader sequence. The term" nucleic acid sequence encoding a mature Fim polypeptide" refers to a sequence without the Fim leader or signal sequence.

As used herein, the term "molecule of interest (MOI)" refers to molecules, either on nucleic acid level, also referred to as gene of interest, GOI, or amino acid level, also referred to as POI, protein of interest. In particular, the MOI is a (poly-)peptide or protein to be expressed in the host cell.

The present inventors recognized that the Fim polypeptide represents a suitable means for promoting expression and delivery of the MOI expressed in a prokaryotic cell, in particular bacteria, to the surface of said bacterial cell or allowing release from the bacterial cell, thus, overcoming the disadvantage known in the art, like aggregation of the recombinantly expressed MOI in inclusion bodies or enrichment in the periplasmatic space.

Hence, another embodiment of the present invention relates to a vector nucleic acid sequence for expressing at least one molecule of interest in a prokaryotic cell and on the surface of the said cell and/or ,eventually, released from said cells in frame from 5' to 3' a nucleic acid encoding a leader sequence, a nucleic acid sequence encoding a molecule of interest and a nucleic acid sequence encoding a mature Fim polypeptide, optionally, having a linker nucleic acid sequence located between the nucleic acid sequence encoding a molecule of interest and the nucleic acid sequence encoding a mature Fim polypeptide wherein the Fim polypeptide is Fim H, A, F, or G, in particular Fim H. For example, the linker is at least partly composed of parts of the multiple cloning sites. In addition, the linker nucleic acid sequence may encode elements or means allowing separating the expressed molecule of interest from the Fim moiety of the expressed fusion protein. The vector nucleic acid according to present invention allows the expression of a fusion protein comprising the Fim polypeptide moiety and a moiety of the molecule of interest, optionally, separated by a linker moiety. Typically, the molecule of interest is a protein or peptide.

In an embodiment of the present invention, the vector nucleic acid includes a linker nucleic acid sequence present in frame between the nucleic acid sequence encoding the molecule of interest the nucleic acid sequence en-codingand the mature Fim polypeptide wherein the Fim polypeptide is Fim H, A, F, or G, in particular Fim H. For example, the linker nucleic acid sequence encodes a cleavage site allowing separation of the Fim polypeptide and the molecule of interest after expression. Alternatively, or in addition, the linker nucleic acid sequence may be arranged at the 5' end of the nucleic acid sequence allowing purification of the fusion protein of the Fim polypeptide and the molecule of interest. The skilled person is well aware of the order of elements present in the vector nucleic acid. Usually, the order is given from the 5' to the 3' of the nucleic acid sequence. That is, the nucleic acid sequence encoding the molecule of interest is located upstream of the mature Fim nucleic acid sequence and downstream of the leader sequence. If present, the multiple cloning site is at the 5' end of the nucleic acid sequence encoding the mature Fim polypeptide wherein the Fim polypeptide is Fim H, A, F, or G, in particular Fim H. That is, the order for 5' to 3' is a nucleic acid encoding a leader sequence, MOI, optionally a linker, and the mature FimH wherein the Fim polypeptide is Fim H, A, F, or G, in particular Fim H. Further, a tag may be present allowing purification of the fusion protein after expression or of the MOI after expression and cleavage.

In an embodiment of the present invention, the vector nucleic acid contain further a nucleic acid sequence operable linked with the nuclear acid sequence encoding the molecule of interest and the nuclear acid sequence encoding the Fim polypeptide wherein the Fim polypeptide is Fim H, A, F, or G, in particular Fim H. Said nuclear acid sequence encodes a tag molecule. The tag molecule may be located at the 5' end or 3' end of the vector nucleic acid and, accordingly, the N-terminal end and/or C-terminal end of the polypeptide. In some embodiments, the tag moiety is present downstream of the nucleic acid sequence encoding the molecule of interest and the nucleic acid sequence encoding the Fim polypeptide.

The skilled person is well aware of suitable tag molecules including peptide tags like FLAG-tag, HA-tag, His-tag, Myc-tag, S-tag SBP-tag, Softag 1, Softag 3, TC tag, V5 tag, Xpress tag.

In an embodiment, the leader sequence is a leader sequence of Fim genes., like the Fim Protein, e.g. of the Fim H protein.

In a further embodiment, the nucleic acid sequence according to the present invention comprise further elements including at the 5' end of said nucleic acid sequence an inducible promoter operably linked with leader sequence. Said inducible promoter allows transient expression of the nucleic acid comprising the coding sequence for the MOI and Fim.

The skilled person is well aware of suitable promoter sequence being inducible, in particular, being inducible in bacterial cells. For example, the promoter of the vector nucleic acid may be arrived from any known promoter/enhancer system described in the art. Preferably, the promoter/enhancer region is derived from the T7 RNA polymerase promoter to direct high-level expression of the Fim polypeptide and the molecule of interest.

In addition, the vector nucleic acid sequence contains further elements for expression, for example a Shine-Dalgarno-sequence. The promoter may be derived from the LAC operator or the T7 promoter for example regulated by the LAC operator. The skilled person is well aware of suitable promoter and enhancer systems as well as of suitable regulators of the expression in bacterial cells. Furthermore, the vector nucleic acid contains termination elements as described in the art.

If required, suitable selectable markers are present in the vector nucleic acid as well.

That is, in an embodiment of the present invention, the vector nucleic acid is a vector nucleic acid in the order from the 5' end to the 3' end of a 5'-promoter, a nucleic acid encoding a leader sequence, a nucleic acid sequence encoding the molecule of interest, optionally a linker sequence, the nucleic acid sequence encoding a Fim polypeptide wherein the Fim polypeptide is Fim H, A, F, or G, in particular Fim H, and a transcription termination sequence and translation termination sequence.

For example, the nucleic acid sequence encoding the leader sequence is a sequence encoding an amino acid sequence according to Seq. ID No. 1. Further, the nucleic acid encoding the mature Fim polypeptide is a nucleic acid sequence encoding the amino acid sequence according to Seq. ID No.2. The nucleic acid sequence encoding the molecule of interest is a nucleic acid sequence encoding a polypeptide, peptides or proteins of mammalian origin including human.

In another embodiment, the vector nucleic acid is a vector nucleic acid wherein the molecule of interest being secreted extracellularly or being expressed on the surface of the bacterial cell, and having its biological activity. The fusion protein of the molecule of interest and Fim wherein the Fim polypeptide is Fim H, A, F, or G, in particular Fim H is located on the surface of the bacterial cells, thus, the fusion protein may be isolated from the surface of the bacterial cells or, alternatively, be obtained from the supernatant of the bacterial culture wherein said fusion proteins are sheded or secreted. In an embodiment, the fusion protein of the MOI and Fim, wherein the Fim polypeptide is Fim H, A, F, or G, in particular Fim H, or the MOI may be sheded or cleaved into the supernatant based on appropriate cleavage sites present in linker moieties. The skilled person is well aware of suitable systems allowing cleavage between the MOI and Fim or cleavage of MOI and Fim accordingly. A suitable linker sequence including a HRV 3C protease cleavage site is shown in Seq. ID No. 3 and Seq ID NO. 4.

The Fim polypeptide encoded in the vector nucleic acid according to present invention is selected from Fim H, Fim A, Fim F and/or Fim G. In a preferred embodiment, the Fim polypeptide is Fim H. The leader sequence may be a sequence derived from Fim H, Fim A, Fim F and/or Fim G. In a preferred embodiment, the Fim polypeptide is Fim H.

In another embodiment, the present invention relates to a bacterial cell containing a vector nucleic acid according to the present invention wherein said vector nucleic acid may be stably integrated into the host genome or alternatively, or additionally, may be present as plasmids in the bacterial cell. The cell may stably express the polypeptide encoded by the vector nucleic acid or may transiently express the same.

For example, in case of transient expression, the vector nucleic acid contains an inducible promoter allowing transient expression accordingly.

The vector nucleic acid may be introduced into the cells, i.e. the bacterial cells may be transformed with the vector nucleic acid, by known methods.

In a further aspect, the invention relates to the isolated recombinant protein containing at least one module being a Fim polypeptide, wherein the Fim polypeptide is Fim H, A, F, or G, in particular Fim H, and a second module being a molecule of interest whereby the MOI being at the N-terminus of the mature Fim polypeptide.

"Isolated" in the context of the present invention with respect to polypeptides means that the material is removed from its original environment like the cells used to recombinantly produce the polypeptides disclosed herein. These peptides could be part of a composition and still be isolated in that such vector or composition is not part of its natural environment. The polypeptides or protein according to the present invention are preferably provided in an isolated form, and preferably are purified to homogeneity.

The terms polypeptide, peptide and protein are used herein interchangeably unless otherwise identified.

In an embodiment, the isolated recombinant protein according to present invention is obtainable by expression thereof using the vector nucleic acid molecule according to the present invention. That is, the isolated recombinant protein is an expression product of the vector nucleic acid according to the present invention. The term "expression product" means that the polypeptide or protein is the natural translation product of the gene and any nucleic acid sequence coding equivalents resulting from genetic code degeneracy and, thus, coding for the same amino acid(s).

It is clear to the skilled person that the nucleic acid sequence encoding the Fim polypeptide and/or the nucleic acid sequence encoding the molecule of interest include coding equivalents from genetic code degeneracy but encoding for the same amino acids. This is particularly true for the MOI where the nucleic acid sequence encoding the MOI may be adapted to the genetic code of the bacterial cell wherein said nucleic acid sequence is used and is translated into the peptide accordingly.

In an embodiment of the present invention, the nucleic acid sequence encoding the Fim polypeptide is obtained from the bacterial species used as the host for protein expression using the vector nucleic acid according to the present invention. Other embodiments include Fim polypeptides and nucleic acid sequences encoding said Fim polypeptides wherein the Fim is originated from other bacterial species.

Furthermore, the present invention relates to a method for the production of the molecule of interest. Said method comprises a step of transforming a prokaryotic host cell, in particular a bacterial cell, with a vector nucleic acid according to the present invention or using a cell containing the vector according to the present invention.

In an embodiment of said method for the production of the MOI, said method comprises further the step of introducing the nucleic acid sequence encoding the MOI into the vector nucleic acid according to the present invention comprising a nucleic acid sequence encoding a Fim polypeptide wherein the Fim polypeptide is Fim H, A, F, or G, in particular Fim H and a multiple cloning site suitable for introduction of the nucleic acid sequence encoding a molecule of interest within the reading frame of the Fim polypeptide. That is, the multiple cloning site is located between the leader sequence and the mature Fim polypeptide wherein the Fim polypeptide is Fim H, A, F, or G, in particular Fim H. Said step is conducted before transforming the bacterial cell with said vector nucleic acid. The skilled person is well aware of suitable methods and means for introducing said nucleic acid sequence including the molecule of interest into the multiple cloning site of the vector nucleic acid whereby the nucleic acid sequence encoding the MOI is in the reading frame of the Fim polypeptide wherein the Fim polypeptide is Fim H, A, F, or G, in particular Fim H.

In an embodiment of said method for the production of the MOI, the recombinant protein produced in the host cell, namely, the bacterial cell is released by or expressed on the surface of said transformed host cell.

The fusion protein according to present invention comprising the Fim moiety and the MOI moiety, for example in form of the Fim H and the MOI moiety are part of the Typ-I pili on the surface of the bacterial cell. Such modified bacteria can be used for screening for a MOI with particular properties, e.g. for binding or enzymatic activities. In such an embodiment, transformed bacteria are incubated on surfaces or beads coated with the ligand for the MOI using standard binding techniques ("display" or "panning") and bound bacteria are analysed with respect to the Fim H fusion protein to identify the MOI. In case of enzymatic activities which are characteristic for the MOI, transformed bacteria are incubated on plates or membranes coated with the substrate and the Fim H fusion protein of bacteria colonies which metabolize the substrate are further analysed with respect to the MOI.

In a further embodiment, the method for the production of the MOI comprises further steps of culturing the transformed bacterial cells in a culture medium, separating the bacterial cells by culture on the selective culture medium and purifying the MOI from the cell free culture medium. Alternatively or additionally, the bacterial cells are treated by known means to separate the fusion protein or the MOI from the surface of the bacterial cells.

In another embodiment, the present invention relates to a ell containing a vector nucleic acid according to the present invention. In an embodiment of the present invention, this cell is a procaryontic cell, preferentially bacteria, preferentially gram-negative bacteria. For example, the cell is a bacteria, like a gram-negative bacteria conventionally used for protein expression.

In another embodiment, the cell is a protobacteria, preferentially E.coli, Salmonella, Shigella, or enterobacteria, cocci, cyanobacteria, bacilli.

Finally, the present invention provides a kit or system containing the vector nucleic acid according to the present invention, or a cell according to the present invention. The kit or system according to the present invention is particularly useful for the production of recombinant molecules of interest, in particular, molecules of interest having a natural folding structure and the same biological activity of the natural molecule of interest.

The kit or system according to the present invention may contain additionally prokaryotic cells, preferentially bacterial cells, to be used for transformation. Further, the kit or system may contain suitable reagents including buffer and culture medium for transforming the said host cells and for culturing the same.

Moreover, the kit or system may contain additionally means for allowing separation and isolation of the fusion protein or the MOI alone, e.g. based on the tag moiety present in the recombinant protein.

A main advantage of the MOI, typically in form of a polypeptide or protein, also identified as a protein of interest or polypeptide of interest (POI), is its natural folding structure. In addition, the biological activity of the MOI, e.g. in form of the POI, is more or less identical with the biological activity of the natural molecule of interest. So far, no systems are described allowing expression of MOI on the surface of bacterial cells or the production of secreted MOI having a natural folding structure and the same biological activity. The present invention overcomes the problems known in the art in view of enrichment in the periplasm of the bacterial cells or in the inclusion bodies of the bacterial cells or similar procedures

### Molecule of interest or Protein of Interest:

The expression system can be used for producing any protein or peptide, including but not limited to production of diagnostic and therapeutic proteins, such as hormones, growth factors, cytokines, chemokines, coagulation factors, antibodies. The system can be used for the expression of recombinant proteins of various evolutionary origin, such as toxins, vaccines, proteases, oxidoreduc-tases, transferases, hydrolases, lyases, isomerases, ligases, biolumienscence proteins such as luciferases from *Photinus pyralis, Luciola cruciata, Luciola italiaca, Luciola lateralis, Luciola mingrelica, Photuris pennylvanica, Pyrophorus plagiphthalamus, Phrixothrix hirtus, Renilla reniformis, Gaussia princeps, Cypridina noctiluca, Cypridina hilgendorfii, Metridia longa, Oplophorus gracilostris,* autofluorescence proteins, such as wildtype-GFP, EGFP, GFPuv, mCherry, DSRed-monomer (Clontech), mOrange, Venus EYFP, ECYP (Clontech), mCFP, Cerulan, EBFP..

In an embodiment of the present invention, the MOI is a toxin, in particular, a cytotoxic component. For example, the MOI is a moiety being cytotoxic to mammalian cells, in particular, tumour cells. In another embodiment, the MOI is a binding molecule including an antibody or an antibody fragment, like a functional scFv or a Fab. These antibodies and antibody fragments may be used in pharmaceuticals. In a further embodiment, the MOI is an antigen useful in a vaccine. For example, the antigen is a vaccine useful in humans and animals. Suitable antigens are derived from virus mediated disease including hepatitis or influenca as well as vaccines useful *inter alia* in cancer therapy.

### Examples

### Expression of the FimH-GaussiaLuc fusion protein on the surface of BL21-Gold (DE3) E.coli.

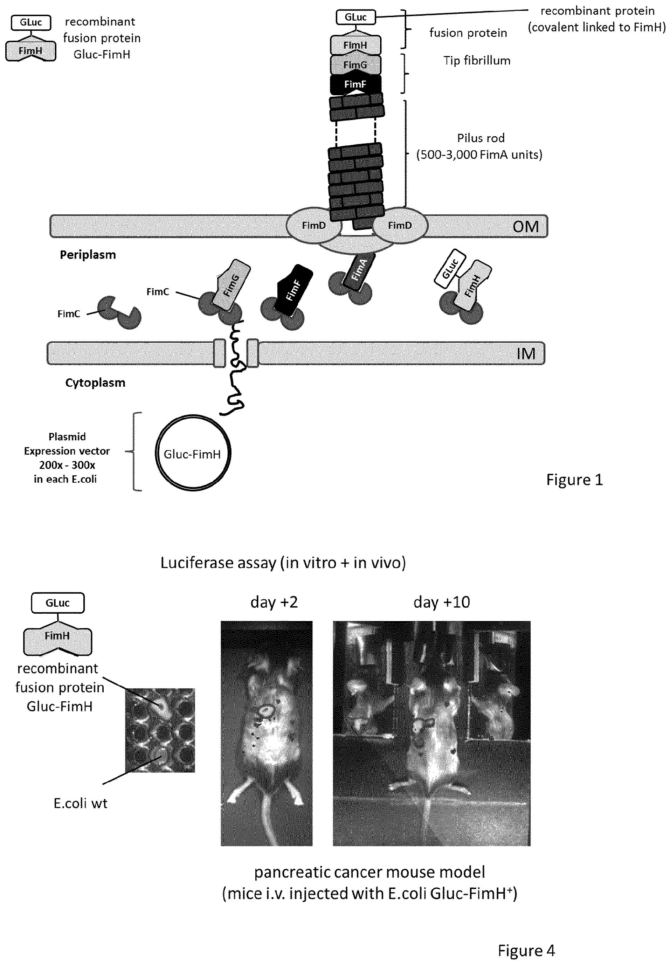

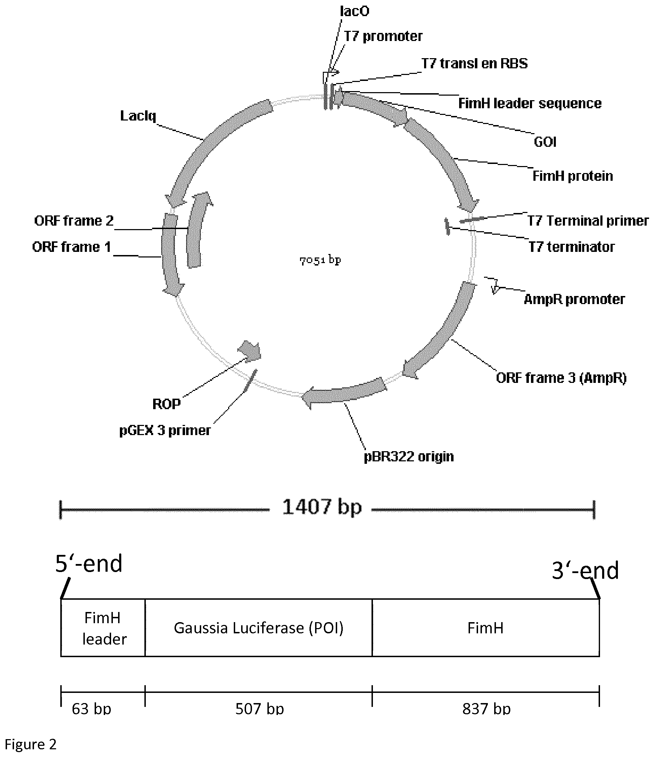

To produce the MOI GaussiaLuc, the cDNA coding for leader FimH-GaussiaLuc-FimH (see Fig.2) was amplified by PCR (polymerase chain reaction) and cloned into the T7 expression vector pET-11d (Agilent Technologies, Stratagene Products Division, La Jolla, CA 92037, US) using the restriction sites Ncol and BamHI. The pET expression vector, derived from the pBR322 plasmid, is engineered to take advantage of the featuers of the T7 bacteriophage gene 10 that promotes high-level transcription and translation. The integration of the lac operator between the T7 promoter and translation initiation sequences allows IPTG-mediated de-repression of the T7 promoter in addition to IPTG-induction of T7 polymerase from *lacUV5* promoter in the DE3 containing bacterial strains (*E.coli* BL21-derived high expression strains). The BL21 strain is generally suitable for protein expression due to its deficiency in *Ion* protease as well as *ompT* outer membrane protease that can degrade proteins. The originally gene 10 leader sequence was replaced by FimH leader sequence due to the intended destination of the expressed protein of interest on the bacterial surface. Fig. 1 shows a model of the synthesis of the Gluc-FimH expression in bacterial cells.

### Inducible Expression of the p-ET11d-GaussiaLuc-FimH vector on the bacterial surface.

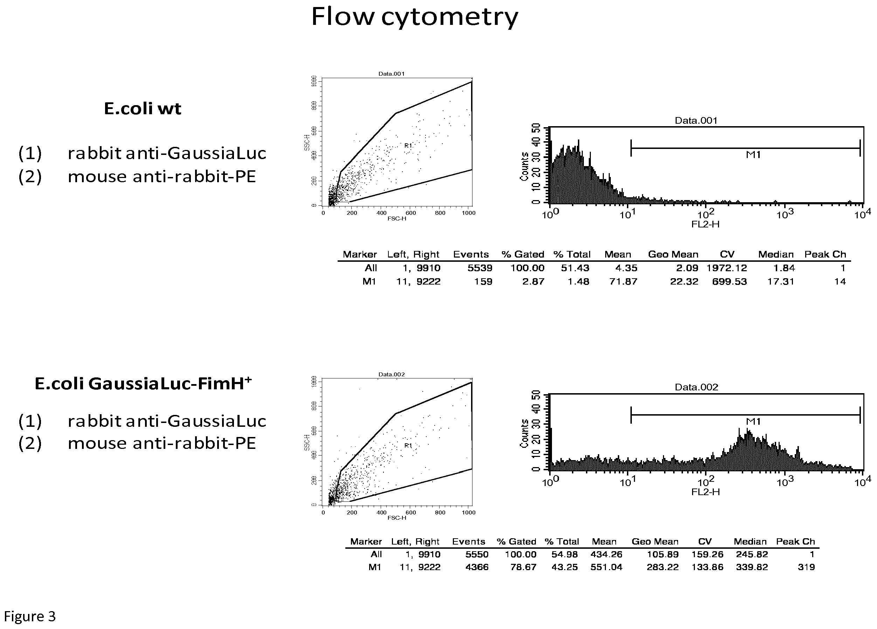

The BL21-Gold expression strain BL21-Gold (DE3) pLysS (Agilent technologies) was transformed with the p-ET11d-GaussiaLuc-FimH vector. Expression of GaussiaLuc-FimH was induced by IGPT administration and analysed by flow cytometry. Therefore, BL21-Gold E.coli with GaussiaLuc-FimH expression were co-incubated with GaussiaLuc-specific rabbit serum [1µg/ml] and with the PE-conjugated rabbit IgG1-specific antibody. Non-transformed BL21-Gold E.coli served as control. Bacteria transformed with the p-ET11d-GaussiaLuc-FimH vector express the GaussiaLuc protein on the surface as detected by a specific antibody. Fig.3.

To demonstrate that the GaussiaLuc protein is functional, transformed bacteria were incubated with benzyl-coelenterazine, which is a substrate for Gaussia luciferase, and light emission was recorded using a Biospace imaging device Biospace Lab, Paris). E.coli bacteria transformed with the p-ET11d-GaussiaLuc-FimH vector show light emission which is not the case with non-transformed E.coli bacteria (Fig.4, left side).

To demonstrate activity of the GaussiaLuc protein expressing bacteria in vivo, transformed bacteria were injected into the tail vein of mice which harbor a transplanted pancreatic cancer in the pancreas upon panc02 cell transplantation. Bioluminescence imaging to detect the GaussiaLuc bacteria was performed upon intra-peritoneal injection of benzyl-coelenterazine (100 µg/mouse or 5 µg/ml) (PJK GmbH, Kleinblittersdorf, Germany) as substrate for the GaussiaLuc. Accumulated transformed bacteria were visualized using the Biospace Imager (Biospace Lab, Paris, France). The light signal indicates GaussiaLuc activity at the site of pancreatic cancer and affected lymph nodes in treated mice. Non-modified bacteria did not produce a light signal (Fig. 4, right side).

### Reference example

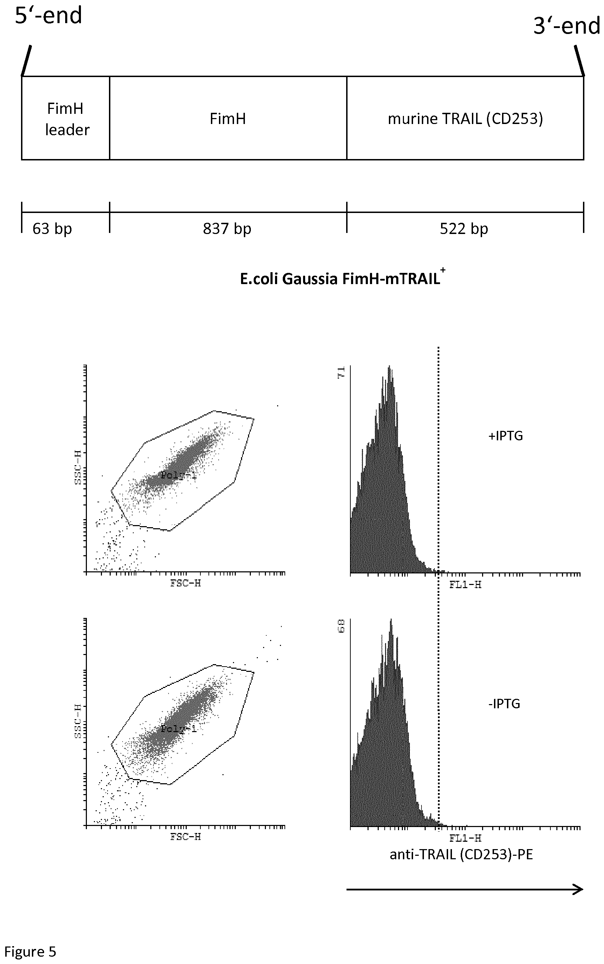

To demonstrate that the expression of the POI on the bacterial surface depends on the integration site of the FimH molecule in the fusion protein, a nucleic acid comprising the expression cassette as shown in Figure 5 has been obtained. Therein, the protein of interest, here the murine protein M trail (CD253) was fused at the 3'-end of the E.coli FimH sequence. The bacteria were transformed as described above with the expression plasmid and the expression of the fusion protein was induced by adding IPTG (1mM) as described above. The expression was detected by flow cytometry using a PE conjugated, murine trail specific rabbit antibody (1mg/ml). As demonstrated in the lower figure of figure 5, no expression was observed on the surface of E.coli for the FimH trail fusion protein. That is, in contrast to the expression of the POI on the surface of E.coli where the FimH moiety was at the C-terminus, expressing the FimH N-terminally to the POI does not result in the expression of the surface. Thus, the order of the moieties present in the fusion protein is of importance.

### Fusion protein containing a linker moiety with a cleavage site

Next, it was demonstrated that the recombinant protein expressed by vector nucleic acid according to the present invention can be cleaved specifically, thus, releasing the POI maintaining its biological activity, namely, being functional.

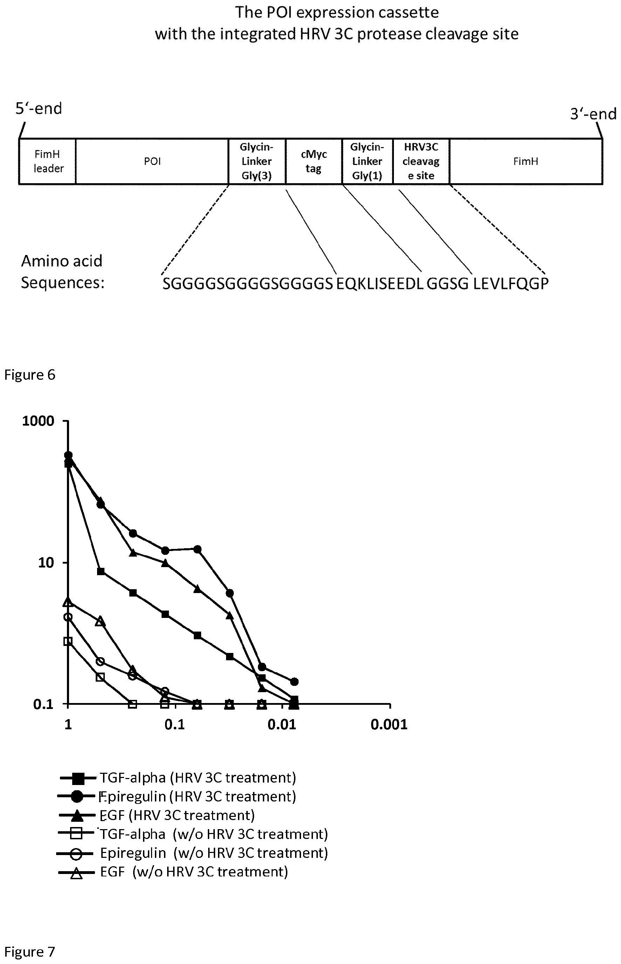

A vector nucleic acid was obtained containing the POI expression cassette as shown in figure 6 containing an integrated HRV 3C protease cleavage site. That is, the sequence of Seq. ID NO. 4 encoding the amino acid sequence of Seq ID. NO. 3 was introduced as a linker nucleic acid sequence located to a nucleic acid sequence encoding a molecule of interest and the nucleic acid sequence encoding a molecule of interest and the nucleic acid sequence encoding a major Fim polypeptide. As shown, the linker sequence consists of a glycin-linker wherein a c-mxc tag is integrated and, thereof at the 3'- and, a HRV 3C cleavage site has been incorporated.

E.coli were transformed with the respective plasmids encoding the protein of interest tagged by a c-myc tag and linked to FimH as described above. Expression was induced by incubation with IPTG as described. Incubation without IPTG served as a control. The expression of the POI was recorded by flow cytometry using a mouse anti-myc antibody an a FITC labelled anti-mouse IgG antibody. It was shown by flow cytometry, that the respective recombinant protein was expressed on the surface of the E.coli after IPTG incubation while when no IPTG was present, the number of positive cells were significantly less.

To demonstrate that the transformed E.coli with the plasmid encoding the protein of interest tagged by c-myc tag and linked to FimH containing the HRV 3C protease cleavage site allow separation of the POI tagged by the c-myc tag after cleaving with the respective protease, the expression of the recombinant proteins of interest was induced by incubation with 1 mM IPTG NLB medium for two hours. In this connection, three different transformed E.coli strains were produced wherein the POI was EGF, TGFα and Epiregulin respectively. All plasmids contain the linker sequence as shown in figure 6.

After incubation, the samples were centrifuged and the supernatant was discarded, the bacteria were resuspended in 1 ml PBAS and treated with HRV 3C protease (10 U) overnight at 4 °C in order to cleave the POI from the FimH part of the fusion protein. In one part of each bacteria culture was incubated overnight without HRV 3C protease and served as a control. The detection of the proteins of interest, namely, EGF, TGFα and Epiregulin, all are EGFR ligands, was performed by ELISA using human EGF, human TGFα and human Epiregulin ELISA kits obtainable from CUSABIO BIOTECH according to the manufacturer's instructions. Dilution level 1 as shown in figure 6 represents 100 µl of undiluted supernatant. As shown in figure 6, release of the recombinant proteins of interest into the supernatant is achieved. Significant amounts of the POI is released in the supernatant.

As shown, HRV 3C treatment of E.coli expressing the recombinant protein on the surface shows release of the respective POI into the supernatant while the control cultures without HRV 3C treatment a small amount of the POI is released.

Further, POI produced in the E.coli are functional. That is, the POI released after cleavage remain their biological activity and are functional. In this connection, the supernatants containing the EGFR ligands, namely EGF, TGF TGFα or Epiregulin, respectively, were co-incubated (50 µl/1x10⁶ cells) with cells of the line 293T, which express the EGF receptor. Using the intrasure kit of Bacton Dickinson, the phosphorylation ot the EGF receptor at tyrosine 1173 was detected by flow cytometry using goat anti p-EGFR (Tyr 1173) antibody and APC-labeled anti-goat IgG antibody. Binding of EGF, Epiregulin or TGFα to the EGF receptor activates tyrosine-specific protein kinase activity of the EGF receptor. The carboxy terminal tyrosine residues on EGFR, Tyr 1068 and Tyr 1173, are the major sites of autophosphorylation which occurs as a result of EGF binding. Once activated, EGFR mediates the binding of the phosphotyrosine binding domain of GRB2 through direct interactions with Tyr 1068 and Tyr 1086 and through indirect interactions of Tyr 1173 with the Ras signalling pathway. Tyr 1173 of EGFR also functions as a kinase substrate. The data obtained (not shown) identifies that the E.coli produce POI according to the present invention are functional and allow induction of phosphorylation of the EGF receptor.

## Claims

1. A vector nucleic acid suitable for expressing at least one molecule of interest in a prokaryotic cell, in particular bacterial cell, and on the surface of said cell, or released by the said cell comprising in frame from 5' to 3': a nucleic acid sequence encoding a leader sequence, a multiple cloning site for introduction of the nucleic acid sequence encoding a molecule of interest, and a nucleic acid sequence encoding a mature Fim polypeptide, wherein the Fim polypeptide is Fim H, A, F, or G, in particular Fim H.

2. A vector nucleic acid suitable for expressing at least one molecule of interest in a prokaryotic cell, in particular, a bacterial cell, and on the surface of a said cell or released by said cell comprising
a nucleic acid sequence encoding in frame from 5' to 3': a nucleic acid sequence encoding a leader sequence, a nucleic acid sequence encoding a molecule of interest and, a nucleic acid sequence encoding a mature Fim polypeptide, optionally, having a linker nucleic acid sequence located between the nucleic acid sequence encoding a molecule of interest and the nucleic acid sequence encoding a mature Fim polypeptide, wherein the Fim polypeptide is Fim H, A, F, or G, in particular Fim H.

3. The vector nucleic acid according to claim 2 wherein a linker nucleic acid sequence is present in frame between the nucleic acid sequence encoding the molecule of interest and the nucleic acid sequence encoding the mature Fim polypeptide, in particular, wherein the linker nucleic acid sequence encodes a cleavage site allowing separation of the Fim polypeptide and the molecule of interest after expression.

4. The vector nucleic acid according to any one of claims 2 to 3 further containing a nucleic acid sequence operably linked with the nucleic acid sequence encoding the molecule of interest and the nucleic acid sequence encoding the Fim polypeptide whereby said nucleic acid sequence encodes a tag molecule.

5. The nucleic acid sequence according to any one of the preceding claims comprising at the 5' end of said nucleic acid sequence an inducible promoter operably linked with said nucleic acid sequence encoding the leader sequence.

6. The vector nucleic acid according to any one of the preceding claims whereby the molecule of interest having its biological activity being released extracellulary or being expressed on the surface of the prokaryotic cell.

7. The vector nucleic acid according to any one of the preceding claims wherein the leader sequence is a Fim leader sequence, in particular, a FimH leader sequence.

8. Cell containing a vector nucleic acid as defined in any one of claims 1 to 7 wherein said vector nucleic acid may be i) stably integrated into the host genome or episomally replicating or ii) may be present as a minicircle expression vector for stable or transient expression, and, preferably, wherein the cell is a procaryontic cell, preferentially bacteria, like gram-negative bacteria.

9. Isolated recombinant protein containing at least one module being a mature Fim polypeptide, wherein the Fim polypeptide is Fim H, A, F, or G, in particular Fim H and a second module being a molecule of interest whereby the molecule of interest being located at the N-terminus of the mature Fim polypeptide, optionally, whereby a linker is located between the molecule of interest and the mature Fim polypeptide, in particular, obtainable by expression thereof using a vector nucleic acid according to any one of claims 1 to 7.

10. A method for the production of a molecule of interest comprising the step of transforming a prokaryotic cell, in particular, a bacteria, with the vector nucleic acid as defined in any one of claims 1 to 7, optionally comprising further the step of introducing the nucleic acid sequence encoding the molecule of interest into the vector nucleic acid according to claim 1 before transforming the prokaryotic cell with said vector nucleic acid, in particular, for obtaining a recombinant protein secreted by or expressed on the surface of the transformed cell.

11. The method for the production of a molecule of interest according to claim 10 further comprising the steps of culturing the transformed bacterial cells in a culture medium, optionally, separating the intact bacterial cells and the culture medium, and purifying the molecule of interest from the cell free culture medium.

12. The method according to any one of claims 10 to 11 further comprising the step of cleaving the recombinant protein comprising the molecule of interest and the mature Fim polypeptide at a cleavage site present in linker located between the molecule of interest and the mature Fim polypeptide.

13. A kit or system containing a vector nucleic acid as defined in any one of claims 1 to 7 or a cell according to claim 8.

14. The use of a kit or system according to claim 13 for the production of recombinant molecules of interest, in particular, of molecules of interest having a natural folding structure and essentially the same biological activity as the natural molecule of interest.

## Patentansprüche

1. Eine Vektor-Nukleinsäure geeignet zur Expression mindestens eines interessierenden Moleküls in einer prokariotischen Zelle, insbesondere in einer Bakterienzelle, und auf der Oberfläche dieser Zelle, oder freigesetzt durch diese Zelle, umfassend im Leserahmen von 5' zum 3': eine Nukleinsäuresequenz kodierend eine Leader-Sequenz, eine multiple Klonierungsstelle zum Einbringen einer Nukleinsäuresequenz kodierend für das interessierende Molekül, und eine Nukleinsäuresequenz kodierend ein reifes Fim Polypeptid, wobei das Fim Polypeptid ein Fim H, A, F, oder G ist, insbesondere Fim H.

2. Vektor Nukleinsäure geeignet zur Expression mindestens eines interessierenden Moleküls in einer prokariotischen Zelle, insbesondere in einer Bakterienzelle, und auf der Oberfläche dieser Zelle, oder freigesetzt durch diese Zelle umfassend eine Nukleinsäuresequenz kodierend im Leserahmen von 5' zu 3': eine Nukleinsäuresequenz kodierend eine Leadersequenz, eine Nukleinsäuresequenz kodierend ein interessierendes Molekül und eine Nukleinsäure kodierend ein reifes Fim Polypeptid, ggf. mit einer Linker-Nukleinsäuresequenz, die sich zwischen der Nukleinsäuresequenz kodierend das interessierende Molekül und der Nukleinsäuresequenz kodierend das reife Fim Polypeptid befindet, wobei das Fim Polypeptid ein Fim H, A, F, oder G ist, insbesondere Fim H.

3. Die Vektor-Nukleinsäure nach Anspruch 2, wobei die Linker-Nukleinsäuresequenz im Leserahmen zwischen der Nukleinsäuresequenz kodierend das interessierende Molekül und der Nukleinsäuresequenz kodierend das reife Fim Polypeptid, vorliegt, insbesondere, wobei die Linker-Nukleinsäuresequenz eine Schnittsttelle kodiert, die eine Trennung des Fim Polypeptids und des interessierenden Moleküls nach Expression erlauben.

4. Die Vektor-Nukleinsäure nach einem der Ansprüche 2 oder 3, weiterhin enthaltend eine Nukleinsäuresequenz, die operativ verbunden ist mit der Nukleinsäuresequenz kodierend das interessierende Molekül und der Nukleinsäuresequenz kodierend das Fim Polypeptid, wobei diese Nukleinsäuresequenz ein Tag-Molekül kodiert.

5. Die Nukleinsäuresequenz nach einem der vorigen Ansprüche, umfassend am 5'-Ende dieser Nukleinsäuresequenz einen induzierbaren Promoter, der mit der Leader-Sequenz kodierende Nukleinsäuresequenz operativ verbunden ist.

6. Die Vektor-Nukleinsäure nach einem der vorigen Ansprüche, wobei das interessierende Molekül seine biologische Aktivität aufweist, wenn es extrazellulär freigesetzt wird oder auf der Oberfläche der prokariotischen Zelle exprimiert wird.

7. Vektor-Nukleinsäure nach einem der vorigen Ansprüche, wobei die Leader-Sequenz eine Fim Leader-Sequenz ist, insbesondere eine Fim H Leader-Sequenz.

8. Zelle, enthaltend eine Vektor-Nukleinsäure wie in einem der Ansprüche 1 bis 7 definiert, wobei dieser Vektor-Nukleinsäure i) stabil in das Wirtsgenom integriert oder episomal replizierend sein kann oder ii) als Minizirkel-Expressionsvektor vorliegen kann zur stabilen oder transienten Expression, und, bevorzugt, wobei die Zelle eine prokariotische Zelle, insbesondere Bakterien, wie gram-negative Bakterien sind.

9. Isoliertes rekombinantes Protein enthaltend mindestens ein Modul, das ein reifes Fim Polypeptid ist, wobei das Fim Polypeptid Fim H, A, F, oder G, insbesondere Fim H ist, und ein zweites Modul, das das interessierende Molekül darstellt, wobei das interessierende Molekül sich am N-Terminus des reifen Fim-Polypeptids befindet, ggf., wobei ein Linker zwischen dem interessierenden Molekül und dem reifen Fim-Polypeptid vorliegt, insbesondere, erhältlich durch Expression hiervon unter Verwendung einer Vektor-Nukleinsäure nach einem der Ansprüche 1 bis 7.

10. Verfahren zur Herstellung eines interessierenden Moleküls, umfassend den Schritt des Transformierens einer prokariotischen Zelle, insbesondere von Bakterien, mit der Vektor-Nukleinsäure wie in einem der Ansprüche 1 bis 7 definiert, ggf. umfassend den weiteren Schritt des Einbringens der Nukleinsäuresequenz kodierend das interessierende Molekül in die Vektor-Nukleinsäure nach Anspruch 1 vor Transformieren der prokariotischen Zelle mit der Vektor-Nukleinsäure, insbesondere zum Erhalt eines rekombinanten Proteins sekretiert durch die transformierte Zelle oder auf der Oberfläche der transformierten Zelle exprimiert.

11. Das Verfahren zur Herstellung eines interessierenden Moleküls nach Anspruch 10, weiter umfassend die Schritte des Kultivierens der transformierten Bakterienzellen in einem Kulturmedium, ggf., Trennen der intakten Bakterienzellen und des Kulturmediums und Aufreinigen des interessierenden Moleküls aus dem zellfreien Kulturmedium.

12. Verfahren nach einem der Ansprüche 10 bis 11, weiterhin umfassend den Schritt des Schneidens des rekombinanten Proteins umfassend das interessierende Molekül und das reife Fim-Polypeptid an der Schnittstelle vorliegend in dem Linker, der sich zwischen dem interessierenden Molekül und dem reifen Fim-Polypeptid befindet.

13. Kit oder System enthaltend eine Vektor-Nukleinsäure, wie in einem der Ansprüche 1 bis 7 definiert oder eine Zelle nach Anspruch 8.

14. Verwendung eines Kits oder Systems nach Anspruch 13 zur Herstellung von rekombinanten interessierenden Molekülen, insbesondere von interessierenden Molekülen, die eine natürliche Faltungsstruktur aufweisen und im Wesentlichen die gleiche biologische Aktivität haben wie das natürliche interessierende Molekül.

## Revendications

1. Acide nucléique de vecteur adapté pour exprimer au moins une molécule d'intérêt dans une cellule procaryote, en particulier une cellule bactérienne, et sur la surface de ladite cellule, ou libéré par ladite cellule comprenant, dans le cadre de 5' vers 3' : une séquence d'acide nucléique codant pour une séquence de tête, un site de codage multiple pour l'introduction de la séquence d'acide nucléique codant pour une molécule d'intérêt, et une séquence d'acide nucléique codant pour un polypeptide Fim mature, le polypeptide Fim étant Fim H, A, F ou G, en particulier Fim H.

2. Acide nucléique de vecteur adapté pour exprimer au moins une molécule d'intérêt dans une cellule procaryote, en particulier une cellule bactérienne, et sur la surface de ladite cellule, ou libéré par ladite cellule comprenant une séquence d'acide nucléique codant pour, dans le cadre de 5' vers 3' : une séquence d'acide nucléique codant pour une séquence de tête, une séquence d'acide nucléique codant pour une molécule d'intérêt et, une séquence d'acide nucléique codant pour un polypeptide Fim mature, comportant facultativement une séquence d'acide nucléique de lieur située entre la séquence d'acide nucléique codant pour une molécule d'intérêt et la séquence d'acide nucléique codant pour un polypeptide Fim mature, le polypeptide Fim étant Fim H, A, F ou G, en particulier Fim H.

3. Acide nucléique de vecteur selon la revendication 2, dans lequel une séquence d'acide nucléique de lieur est présente dans le cadre entre la séquence d'acide nucléique codant pour la molécule d'intérêt et la séquence d'acide nucléique codant pour le polypeptide Fim mature, en particulier, dans lequel la séquence d'acide nucléique de lieur code pour un site de clivage permettant la séparation du polypeptide Fim et de la molécule d'intérêt après expression.

4. Acide nucléique de vecteur selon l'une quelconque des revendications 2 à 3, contenant en outre une séquence d'acide nucléique fonctionnellement liée à la séquence d'acide nucléique codant pour la molécule d'intérêt et la séquence d'acide nucléique codant pour le polypeptide Fim de sorte que ladite séquence d'acide nucléique code pour une molécule d'étiquette.

5. Séquence d'acide nucléique selon l'une quelconque des revendications précédentes comprenant, à l'extrémité 5' de ladite séquence d'acide nucléique, un promoteur réductible fonctionnellement lié à ladite séquence d'acide nucléique codant pour la séquence de tête.

6. Acide nucléique de vecteur selon l'une quelconque des revendications précédentes, la molécule d'intérêt ayant son activité biologique libérée de façon extracellulaire ou étant exprimée sur la surface de la cellule procaryote.

7. Acide nucléique de vecteur selon l'une quelconque des revendications précédentes, dans lequel la séquence de tête est une séquence de tête de Fim, en particulier une séquence de tête de FimH.

8. Cellule contenant un acide nucléique de vecteur tel que défini dans l'une quelconque des revendications 1 à 7, dans laquelle ledit acide nucléique de vecteur peut être i) intégré de façon stable dans le génome de l'hôte ou se répliquant de façon épisomique ou ii) peut être présent sous la forme d'un vecteur d'expression de minicercle pour expression stable ou transitoire, et, de préférence, la cellule étant une cellule procaryote, de préférence une bactérie, telle qu'une bactérie Gram négatif.

9. Protéine recombinante isolée contenant au moins un module étant un polypeptide Fim mature, le polypeptide Fim étant Fim H, A, F ou G, en particulier Fim H et un deuxième module étant une molécule d'intérêt, la molécule d'intérêt étant située à l'extrémité N-terminale du polypeptide Fim mature, facultativement, dans laquelle un lieur est situé entre la molécule d'intérêt et le polypeptide Fim mature, en particulier, pouvant être obtenue par expression de celle-ci au moyen d'un acide nucléique de vecteur selon l'une quelconque des revendications 1 à 7.

10. Procédé de production d'une molécule d'intérêt comprenant l'étape de transformation d'une cellule procaryote, en particulier, une bactérie, avec l'acide nucléique de vecteur tel que défini dans l'une quelconque des revendications 1 à 7, comprenant en outre, facultativement, l'étape d'introduction de la séquence d'acide nucléique codant pour la molécule d'intérêt dans l'acide nucléique de vecteur selon la revendication 1 avant la transformation de la cellule procaryote avec ledit acide nucléique de vecteur, en particulier, pour obtenir une protéine recombinante sécrétée par ou exprimée sur la surface de la cellule transformée.

11. Procédé de production d'une molécule d'intérêt selon la revendication 10, comprenant en outre les étapes de culture des cellules bactériennes transformées dans un milieu de culture, facultativement, séparation des cellules bactériennes intactes et du milieu de culture, et purification de la molécule d'intérêt à partir du milieu de culture sans cellules.

12. Procédé selon l'une quelconque des revendications 10 à 11, comprenant en outre l'étape de clivage de la protéine recombinante comprenant la molécule d'intérêt et le polypeptide Fim mature à un site de clivage présent dans le lieur situé entre la molécule d'intérêt et le polypeptide Fim mature.

13. Kit ou système contenant un acide nucléique de vecteur tel que défini dans l'une quelconque des revendications 1 à 7 ou une cellule selon la revendication 8.

14. Utilisation d'un kit ou système selon la revendication 13 pour la production de molécules recombinantes d'intérêt, en particulier, de molécules d'intérêt ayant une structure de repliement naturelle et pratiquement la même activité biologique que la molécule d'intérêt naturelle.


### SEQUENCE LISTING

<110> Universitaet zu Koeln
<120> Vector and method for expressing molecules of interest in a
   bacterial cell
<130> 4677-004 PCT-1
<150> EP14165102.6
   <151> 2015-04-17
<160> 4
<170> PatentIn version 3.5
<210> 1
   <211> 21
   <212> PRT
   <213> Escherichia coli
<400> 1
   Met Val Arg Val Ile Thr Leu Phe Ala Val Leu Leu Met Gly Trp Ser
   1               5                   10                  15
   Val Asn Ala Trp Ser
               20
<210> 2
   <211> 279
   <212> PRT
   <213> Escherichia coli
<400> 2
   Phe Ala Cys Lys Thr Ala Asn Gly Thr Ala Ile Pro Ile Gly Gly Gly
   1               5                   10                  15
   Ser Ala Asn Val Tyr Val Asn Leu Ala Pro Val Val Asn Val Gly Gln
               20                  25                  30
   Asn Leu Val Val Asp Leu Ser Thr Gln Ile Phe Cys His Asn Asp Tyr
           35                  40                  45
   Pro Glu Thr Ile Thr Asp Tyr Val Thr Leu Gln Arg Gly Ser Ala Tyr
       50                  55                  60
   Gly Gly Val Leu Ser Asn Phe Ser Gly Thr Val Lys Tyr Ser Gly Ser
   65                  70                  75                  80
   Ser Tyr Pro Phe Pro Thr Thr Ser Glu Thr Pro Arg Val Val Tyr Asn
                   85                  90                  95
   Ser Arg Thr Asp Lys Pro Trp Pro Val Ala Leu Tyr Leu Thr Pro Val
               100                 105                 110
   Ser Ser Ala Gly Gly Val Ala Ile Lys Ala Gly Ser Leu Ile Ala Val
           115                 120                 125
   Leu Ile Leu Arg Gln Thr Asn Asn Tyr Asn Ser Asp Asp Phe Gln Phe
       130                 135                 140
   Val Trp Asn Ile Tyr Ala Asn Asn Asp Val Val Val Pro Thr Gly Gly
   145                 150                 155                 160
   Cys Asp Val Ser Ala Arg Asp Val Thr Val Thr Leu Pro Asp Tyr Pro
                   165                 170                 175
   Gly Ser Val Pro Ile Pro Leu Thr Val Tyr Cys Ala Lys Ser Gln Asn
               180                 185                 190
   Leu Gly Tyr Tyr Leu Ser Gly Thr Thr Ala Asp Ala Gly Asn Ser Ile
           195                 200                 205
   Phe Thr Asn Thr Ala Ser Phe Ser Pro Ala Gln Gly Val Gly Val Gln
       210                 215                 220
   Leu Thr Arg Asn Gly Thr Ile Ile Pro Ala Asn Asn Thr Val Ser Leu
   225                 230                 235                 240
   Gly Ala Val Gly Thr Ser Ala Val Ser Leu Gly Leu Thr Ala Asn Tyr
                   245                 250                 255
   Ala Arg Thr Gly Gly Gln Val Thr Ala Gly Asn Val Gln Ser Ile Ile
               260                 265                 270
   Gly Val Thr Phe Val Tyr Gln
           275
<210> 3
   <211> 38
   <212> PRT
   <213> artificial
<220>
   <223> synthetic peptide
<400> 3
   Ser Gly Gly Gly Gly Ser Gly Gly Gly Gly Ser Gly Gly Gly Gly Ser
   1               5                   10                  15
   Glu Gln Lys Leu Ile Ser Glu Glu Asp Leu Gly Gly Ser Gly Leu Glu
               20                  25                  30
   Val Leu Phe Gln Gly Pro
           35
<210> 4
   <211> 114
   <212> DNA
   <213> artificial
<220>
   <223> synthetic nucleic acid
<400> 4
   agcggcggtg gcggtagcgg cggtggcggt agcggcggtg gcggtagcga acagaaactg      60
   attagcgaag aagatctggg cggtagcggc ctggaagtgc tgtttcaggg tccg           114